# EUROPEAN PATENT APPLICATION

(11) **EP 2 942 062 A1**
(43) Date of publication of application: **11.11.2015**
(21) Application number: 15171722.0
(22) Date of filing: 10.02.2010
(51) Int. Cl.: A61K 39/145, A61K 39/12, A61K 39/00

(54) **INFLUENZA VACCINE REGIMENS FOR PANDEMIC-ASSOCIATED STRAINS**

(30) Priority: 10.02.2009 US 207371 P
(62) Divisional of application: 10704989.2
(71) Applicant: Novartis AG, 4056 Basel (CH)
(72) Inventor: GROTH, Nicola, Siena (IT); FRAGAPANE, Elena, Siena (IT)
(74) Representative: Carpmaels & Ransford LLP

(57) **Abstract**

In contrast to known regimens where pandemic-associated antigens are given 3-4 weeks apart for immunisation, according to the invention two doses of a pandemic-associated antigen are administered to a human 1 week apart, 2 weeks apart or 6 weeks apart. Thus the invention provides a method for immunizing a human, comprising steps of: (a) administering to the human a first vaccine comprising antigen from a pandemic-associated influenza virus strain; and then 1/2/6 week(s) later, (b) administering to the same human a second influenza vaccine comprising antigen from the pandemic-associated influenza virus strain.

## Description

### TECHNICAL FIELD

This invention is in the field of regimens for administering vaccines for protecting against influenza virus infection, and in particular vaccines which include antigens from pandemic-associated strains.

### BACKGROUND ART

Prevailing influenza A viruses are in subtype H1N1 and H3N2, but it is expected that the H5 subtype may become prevalent in the near future. As the human population is immunologically naïve to the new hemagglutinin subtype then this antigenic shift will cause a pandemic outbreak of influenza.

In preparing for an influenza pandemic it has been proposed to use a pre-pandemic vaccination strategy. Patients are immunized with a current H5 strain (from birds) in the hope that the resulting immunity will be useful when the pandemic occurs, despite any antigenic drift that may occur in the meantime. In 2008 GlaxoSmithKline's PREPANDRIX™ product was approved in Europe for human pre-pandemic use.

The PREPANDRIX™ product is administered according to a two dose regimen. Clinical trials administered the doses 21 days apart *(i.e.* at days 0 and 21), and this regimen is also reported in reference 1. References 2 and 3 report that a pre-pandemic vaccine from Sanofi Pasteur was also administered at days 0 and 21. The AFLUNOV™ product from Novartis Vaccines has also been administered by this regimen [4]. Similarly, reference 5 reports a 21 day two dose regimen for a vaccine from CSL in Australia, and the same regimen was used in reference 6. Reference 7 mentions various studies using 21 day two dose regimens, but also mentions a 28 day regimen (see also reference 8). A study in macaques [9] administered two doses at days 0 and 27. In one preclinical study PREPANDRIX™ was administered to rabbits at days 0 and 24.

It is an object of the invention to provide further and improved regimens for administration of pandemic-associated influenza vaccines e.g. for pre-pandemic immunisation.

### DISCLOSURE OF THE INVENTION

In contrast to prior art regimens where doses are given 3-4 weeks apart, according to the invention two doses of a pandemic-associated influenza antigen are administered to a human subject 1 week apart, 2 weeks apart or 6 weeks apart.

Thus the invention provides a method for immunizing a human, comprising steps of: (a) administering to the human a first vaccine comprising antigen from a pandemic-associated influenza virus strain; and then w week(s) later, (b) administering to the human a second influenza vaccine comprising antigen from the pandemic-associated influenza virus strain.

The invention also provides first and second influenza vaccines comprising antigen from a pandemic-associated influenza virus strain, for use in immunizing a human by this method.

The invention also provides the use of first and second vaccines, each comprising antigen from a pandemic-associated influenza virus strain, in the manufacture of medicaments for use in immunizing a human, wherein the first and second vaccines are administered to the same human w week(s) apart.

The invention also provides the use of a first vaccine comprising antigen from a pandemic-associated influenza virus strain, in the manufacture of a medicament for pre-immunizing a human who will receive a second vaccine comprising antigen from the pandemic-associated influenza virus strain w week(s) later.

The invention also provides the use of a second vaccine comprising antigen from a pandemic-associated influenza virus strain, in the manufacture of a medicament for immunizing a human who had received a first vaccine comprising antigen from the pandemic-associated influenza virus strain w week(s) before receiving the second vaccine.

The invention also provides a kit comprising first and second vaccines, each comprising antigen from a pandemic-associated influenza virus strain, wherein the first and second vaccines are for administration to a human w week(s) apart.

The value of *w* is 1, 2 or 6.

### Vaccines

Various forms of influenza virus vaccine are currently available, and vaccines are generally based either on live virus or on inactivated virus. Inactivated vaccines may be based on whole virions, split virions, or on purified surface antigens. Influenza antigens can also be presented in the form of virosomes. The invention can be used with any of these types of vaccine, but will typically be used with inactivated vaccines.

Where inactivated virus is used, the vaccine may comprise whole virion, split virion, or purified surface antigens (including hemagglutinin and, usually, also including neuraminidase). Chemical means for inactivating a virus include treatment with an effective amount of one or more of the following agents: detergents, formaldehyde, β-propiolactone, methylene blue, psoralen, carboxyfullerene (C60), binary ethylamine, acetyl ethyleneimine, or combinations thereof. Non-chemical methods of viral inactivation are known in the art, such as for example UV light or gamma irradiation.

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Split virions are obtained by treating purified virions with detergents (*e.g.* ethyl ether, polysorbate 80, deoxycholate, tri-*N-*butyl phosphate, Triton X-100, Triton N101, cetyltrimethylammonium bromide, Tergitol NP9, *etc.)* to produce subvirion preparations, including the 'Tween-ether' splitting process. Methods of splitting influenza viruses are well known in the art *e.g.* see refs. 10-15, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic *(e.g.* cationic) surfactants *e.g.* alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-*N*-butyl phosphate, Cetavlon, myristyltrimethylammonium salts, lipofectin, lipofectamine, and DOT-MA, the octyl- or nonylphenoxy polyoxyethanols (*e.g.* the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (e.g. in a sucrose density gradient solution). Thus a splitting process can involve clarification of the virion-containing material (to remove non-virion material), concentration of the harvested virions (*e.g*. using an adsorption method, such as CaHPO₄ adsorption), separation of whole virions from non-virion material, splitting of virions using a splitting agent in a density gradient centrifugation step (*e.g.* using a sucrose gradient that contains a splitting agent such as sodium deoxycholate), and then filtration (e.g. ultrafiltration) to remove undesired materials. Split virions can usefully be resuspended in sodium phosphate-buffered isotonic sodium chloride solution. The PREPANDRIX™, BEGRIVAC™, FLUARIX™, FLUZONE™ and FLUSHIELD™ products are split vaccines.

Purified surface antigen vaccines comprise the influenza surface antigens haemagglutinin and, typically, also neuraminidase. Processes for preparing these proteins in purified form are well known. The AFLUNOV™, FLUVIRIN™, AGRIPPAL™ and INFLUVAC™ products are examples.

Another form of inactivated influenza antigen is the virosome [16] (nucleic acid free viral-like liposomal particles). Virosomes can be prepared by solubilization of influenza virus with a detergent followed by removal of the nucleocapsid and reconstitution of the membrane containing the viral glycoproteins. An alternative method for preparing virosomes involves adding viral membraneglycoproteins to excess amounts of phospholipids, to give liposomes with viral proteins in their membrane. The invention can be used to store bulk virosomes. as in the INFLEXAL V™ and INVAVAC™ products. Virosomal H5N1 vaccines are known [17].

The first and second vaccines administered according to a regimen of the invention are preferably of the same type *i.e.* both are inactivated or both are live. When both are inactivated, the type of inactivation is preferably the same *i.e.* both are whole virions, both are split virions, both are virosomes, or both are purified surface antigens. Split virions and surface antigens are preferred.

HA is the main immunogen in current inactivated influenza vaccines, and vaccine doses are standardised by reference to HA levels, typically measured by SRID. Existing seasonal vaccines typically contain about 15µg of HA per strain, although lower doses can be used *e.g.* for children, or in pandemic situations, or when using an adjuvant. Fractional doses such as ½ *(i.e.* 7.5 µg HA per strain), ¼ (*i.e.* 3.75µg HA, as in PREPANDRIX™) and ⅛ have been used, as have higher doses *(e.g.* 3x or 9x doses [18,19]). Except where otherwise stated, vaccines may include between 0.1 and 150µg of HA from the pandemic-associated influenza strain, preferably between 0.1 and 50µg *e.g.* 0.1-20µg, 0.1-15µg, 0.1-10µg, 0.1-7.5µg, 0.5-5µg, *etc.* Particular doses include *e.g.* about 45, about 30, about 15, about 10, about 7.5, about 5, about 3.75, about 1.9, about 1.5, *etc.* µg.

For live vaccines, dosing is measured by median tissue culture infectious dose (TCID₅₀) rather than HA content, and a TCID₅₀ of between 10⁶ and 10⁸ (preferably between 10^{6.5}-10^{7.5}) per strain is typical.

Vaccines of the invention include antigen from a pandemic-associated influenza virus strain. Influenza A virus currently displays sixteen HA subtypes: H1, H2, H3, H4, H5, H6, H7, H8, H9, H10, H11, H12, H13, H14, H15 and H16. Characteristics of a pandemic-associated influenza strain are: (a) it contains a new hemagglutinin compared to the hemagglutinins in currently-circulating human strains, *i.e.* one that has not been evident in the human population for over a decade *(e.g.* H2), or has not previously been seen at all in the human population (*e.g.* H5, H6 or H9, that have generally been found only in bird populations), such that the vaccine recipient and the general human population are immunologically naïve to the strain's hemagglutinin; (b) it is capable of being transmitted horizontally in the human population; and (c) it is pathogenic to humans.

The pandemic-associated influenza virus strain for use with the invention will typically have a H2, H5, H7 or H9 subtype *e.g*. H5N1, H5N3, H9N2, H2N2, H7N1 or H7N7. H5N1 strains are preferred. Within the H5 subtype the strain can be in clade 0, 1, 2, 3, 4, 5, 6, 7, 8 or 9. Pandemic strains can have a H1 subtype (*e.g*. H1N1); for example, the HA can be immunologically cross-reactive with the A/California/04/09 strain.

As described above, vaccines of the invention comprise antigen from a pandemic-associated influenza virus strain. This may be the only antigen in the vaccine, or the vaccine may comprise at least one antigen in addition to the antigen from a pandemic-associated influenza virus strain. The additional antigen(s) may be from influenza virus, and may for example be from influenza A virus and/or influenza B virus. For example, the vaccine may include antigens from a H1N1 strain, a H3N2 strain and/or an influenza B virus strain. 4-valent vaccines of this type are disclosed in ref. 20. Where antigens from two influenza A virus strains are included, these may share a common neuraminidase subtype *e.g*. H1N1 & H5N1. This common N subtype can enhance cross-protection [21].

Strains used with the invention may have a natural HA as found in a wild-type virus, or a modified HA. For instance, it is known to modify HA to remove determinants (*e.g.* hyper-basic regions around the HA1/HA2 cleavage site) that cause a virus to be highly pathogenic in avian species.

An influenza virus used with the invention may be a reassortant strain, and may have been obtained by reverse genetics techniques. Reverse genetics techniques [*e.g*. 22-26] allow influenza viruses with desired genome segments to be prepared *in vitro* using plasmids or other artificial vectors. Typically, it involves expressing (a) DNA molecules that encode desired viral RNA molecules *e.g.* from polI promoters or bacteriophage RNA polymerase promoters, and (b) DNA molecules that encode viral proteins *e.g.* from polII promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA can be used [27-29], and these methods will also involve the use of plasmids to express all or some *(e.g.* just the PB1, PB2, PA and NP proteins) of the viral proteins, with up to 12 plasmids being used in some methods. To reduce the number of plasmids needed, a recent approach [30] combines a plurality of RNA polymerase I transcription cassettes (for viral RNA synthesis) on the same plasmid *(e.g.* sequences encoding 1, 2, 3, 4, 5, 6, 7 or all 8 influenza A vRNA segments), and a plurality of protein-coding regions with RNA polymerase II promoters on another plasmid *(e.g.* sequences encoding 1, 2, 3, 4, 5, 6, 7 or all 8 influenza A mRNA transcripts). Preferred aspects of the reference 30 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using polI promoters to encode the viral RNA segments, it is possible to use bacteriophage polymerase promoters [31]. For instance, promoters for the SP6, T3 or T7 polymerases can conveniently be used. Because of the species-specificity of polI promoters, bacteriophage polymerase promoters can be more convenient for many cell types *(e.g.* MDCK), although a cell must also be transfected with a plasmid encoding the exogenous polymerase enzyme.

In other techniques it is possible to use dual polI and polII promoters to simultaneously code for the viral RNAs and for expressible mRNAs from a single template [32,33].

Thus an influenza A virus may include one or more RNA segments from a A/PR/8/34 virus (typically 6 segments from A/PR/8/34, with the HA and N segments being from a vaccine strain, *i.e.* a 6:2 reassortant). It may also include one or more RNA segments from a A/WSN/33 virus, or from any other virus strain useful for generating reassortant viruses for vaccine preparation. An influenza A virus may include fewer than 6 *(i.e.* 0, 1, 2, 3, 4 or 5) viral segments from an AA/6/60 influenza virus (A/Ann Arbor/6/60). An influenza B virus may include fewer than 6 *(i.e.* 0, 1, 2, 3, 4 or 5) viral segments from an AA/1/66 influenza virus (B/Ann Arbor/1/66). Typically, the invention protects against a strain that is capable of human-to-human transmission, and so the strain's genome will usually include at least one RNA segment that originated in a mammalian *(e.g.* in a human) influenza virus. It may include NS segment that originated in an avian influenza virus.

Strains whose antigens can be included in the compositions may be resistant to antiviral therapy *(e.g.* resistant to oseltamivir [34] and/or zanamivir), including resistant pandemic strains [35].

The influenza virus may be attenuated. The influenza virus may be temperature-sensitive. The influenza virus may be cold-adapted. These three features are particularly useful when using live virus as an antigen.

Particularly useful strains are those that have not been passaged through eggs at any stage between isolation from a patient and replication in a cell culture system, inclusive. MDCK cells can be used exclusively for all steps from isolation to virus replication.

In some embodiments, strains used with the invention have hemagglutinin with a binding preference for oligosaccharides with a Sia(α2,6)Gal terminal disaccharide compared to oligosaccharides with a Sia(α2,3)Gal terminal disaccharide. Human influenza viruses bind to receptor oligosaccharides having a Sia(α2,6)Gal terminal disaccharide (sialic acid linked α-2,6 to galactose), but eggs and Vero cells have receptor oligosaccharides with a Sia(α2,3)Gal terminal disaccharide. Growth of human influenza viruses in cells such as MDCK provides selection pressure on hemagglutinin to maintain the native Sia(α2,6)Gal binding, unlike egg passaging.

To determine if a virus has a binding preference for oligosaccharides with a Sia(α2,6)Gal terminal disaccharide compared to oligosaccharides with a Sia(α2,3)Gal terminal disaccharide, various assays can be used. For instance, reference 36 describes a solid-phase enzyme-linked assay for influenza virus receptor-binding activity which gives sensitive and quantitative measurements of affinity constants. Reference 37 used a solid-phase assay in which binding of viruses to two different sialylglycoproteins was assessed (ovomucoid, with Sia(α2,3)Gal determinants; and pig α₂-macroglobulin, which Sia(α2,6)Gal determinants), and also describes an assay in which the binding of virus was assessed against two receptor analogs: free sialic acid (Neu5Ac) and 3'-sialyllactose (Neu5Acα2-3Galβ1-4Glc). Reference 38 reports an assay using a glycan array which was able to clearly differentiate receptor preferences for α2,3 or α2,6 linkages. Reference 39 reports an assay based on agglutination of human erythrocytes enzymatically modified to contain either Sia(α2,6)Gal or Sia(α2,3)Gal. Depending on the type of assay, it may be performed directly with the virus itself, or can be performed indirectly with hemagglutinin purified from the virus.

In some embodiments influenza strains used with the invention have glycoproteins (including hemagglutinin) with a different glycosylation pattern from egg-derived viruses. Thus the glycoproteins will include glycoforms that are not seen in chicken eggs.

### Cell lines

Manufacture of vaccines for use with the invention can use SPF eggs as the substrate for viral growth, wherein virus is harvested from infected allantoic fluids of hens' eggs. Instead, however, cell lines which support influenza virus replication may be used. The cell line will typically be of mammalian origin. Suitable mammalian cells of origin include, but are not limited to, hamster, cattle, primate (including humans and monkeys) and dog cells, although the use of primate cells is not preferred. Various cell types may be used, such as kidney cells, fibroblasts, retinal cells, lung cells, *etc.* Examples of suitable hamster cells are the cell lines having the names BHK21 or HKCC. Suitable monkey cells are e.g. African green monkey cells, such as kidney cells as in the Vero cell line [40-42]. Suitable dog cells are e.g. kidney cells, as in the CLDK and MDCK cell lines.

Thus suitable cell lines include, but are not limited to: MDCK; CHO; CLDK; HKCC; 293T; BHK; Vero; MRC-5; PER.C6 [43]; FRhL2; WI-38; *etc.* Suitable cell lines are widely available *e.g.* from the American Type Cell Culture (ATCC) collection [44], from the Coriell Cell Repositories [45], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940.

The most preferred cell lines are those with mammalian-type glycosylation. As a less-preferred alternative to mammalian cell lines, virus can be grown on avian cell lines *[e.g.* refs. 46-48], including cell lines derived from ducks (e.g. duck retina) or hens. Examples of avian cell lines include avian embryonic stem cells [46,49] and duck retina cells [47]. Suitable avian embryonic stem cells, include the EBx cell line derived from chicken embryonic stem cells, EB45, EB14, and EB14-074 [50]. Chicken embryo fibroblasts (CEF) may also be used. Rather than using avian cells, however, the use of mammalian cells means that vaccines can be free from avian DNA and egg proteins (such as ovalbumin and ovomucoid), thereby reducing allergenicity.

The most preferred cell lines for growing influenza viruses are MDCK cell lines [51-54], derived from Madin Darby canine kidney. The original MDCK cell line is available from the ATCC as CCL-34, but derivatives of this cell line and other MDCK cell lines may also be used. For instance, reference 51 discloses a MDCK cell line that was adapted for growth in suspension culture ('MDCK 33016', deposited as DSM ACC 2219). Similarly, reference 55 discloses a MDCK-derived cell line that grows in suspension in serum-free culture ('B-702', deposited as FERM BP-7449). Reference 56 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 57 discloses MDCK cell lines with high susceptibility to infection, including 'MDCK.5F1' cells (ATCC CRL-12042). Any of these MDCK cell lines can be used.

Virus may be grown on cells in adherent culture or in suspension. Microcarrier cultures can also be used. In some embodiments, the cells may thus be adapted for growth in suspension.

Cell lines are preferably grown in serum-free culture media and/or protein free media. A medium is referred to as a serum-free medium in the context of the present invention in which there are no additives from serum of human or animal origin. The cells growing in such cultures naturally contain proteins themselves, but a protein-free medium is understood to mean one in which multiplication of the cells *(e.g.* prior to infection) occurs with exclusion of proteins, growth factors, other protein additives and non-serum proteins, but can optionally include proteins such as trypsin or other proteases that may be necessary for viral growth.

Cell lines supporting influenza virus replication are preferably grown below 37°C [58] *(e.g.* 30-36°C, or at about 30°C, 31°C, 32°C, 33°C, 34°C, 35°C, 36°C) during viral replication.

Methods for propagating influenza virus in cultured cells generally includes the steps of inoculating a culture of cells with an inoculum of the strain to be grown, cultivating the infected cells for a desired time period for virus propagation, such as for example as determined by virus titer or antigen expression *(e.g.* between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture *(e.g.* monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture *e.g.* before inoculation, at the same time as inoculation, or after inoculation [58].

In useful embodiments, particularly with MDCK cells, a cell line is not passaged from the master working cell bank beyond 40 population-doubling levels.

The viral inoculum and the viral culture are preferably free from (*i.e*. will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [59]. Absence of herpes simplex viruses is particularly preferred.

### Host cell DNA

Where virus has been grown on a cell line then it is standard practice to minimize the amount of residual cell line DNA in the final vaccine, in order to minimize any oncogenic activity of the DNA.

Thus a vaccine composition prepared according to the invention preferably contains less than 10ng (preferably less than 1ng, and more preferably less than 100pg) of residual host cell DNA per dose, although trace amounts of host cell DNA may be present.

Vaccines containing <10ng *(e.g.* <1ng, <100pg) host cell DNA per 15µg of haemagglutinin are preferred, as are vaccines containing <10ng *(e.g.* <1ng, <100pg) host cell DNA per 0.25ml volume. Vaccines containing <10ng *(e.g.* <1ng, <100pg) host cell DNA per 50µg of haemagglutinin are more preferred, as are vaccines containing <10ng *(e.g.* <1ng, <100pg) host cell DNA per 0.5ml volume.

It is preferred that the average length of any residual host cell DNA is less than 500bp *e.g.* less than 400bp, less than 300bp, less than 200bp, less than 100bp, *etc.*

Contaminating DNA can be removed during vaccine preparation using standard purification procedures *e.g.* chromatography, *etc.* Removal of residual host cell DNA can be enhanced by nuclease treatment e.g. by using a DNase. A convenient method for reducing host cell DNA contamination is disclosed in references 60 & 61, involving a two-step treatment, first using a DNase *(e.g.* Benzonase), which may be used during viral growth, and then a cationic detergent *(e.g.* CTAB), which may be used during virion disruption. Removal by β-propiolactone treatment can also be used.

Measurement of residual host cell DNA is now a routine regulatory requirement for biologicals and is within the normal capabilities of the skilled person. The assay used to measure DNA will typically be a validated assay [62,63]. The performance characteristics of a validated assay can be described in mathematical and quantifiable terms, and its possible sources of error will have been identified. The assay will generally have been tested for characteristics such as accuracy, precision, specificity. Once an assay has been calibrated *(e.g.* against known standard quantities of host cell DNA) and tested then quantitative DNA measurements can be routinely performed. Three main techniques for DNA quantification can be used: hybridization methods, such as Southern blots or slot blots [64]; immunoassay methods, such as the Threshold™ System [65]; and quantitative PCR [66]. These methods are all familiar to the skilled person, although the precise characteristics of each method may depend on the host cell in question *e.g.* the choice of probes for hybridization, the choice of primers and/or probes for amplification, *etc.* The Threshold™ system from *Molecular Devices* is a quantitative assay for picogram levels of total DNA, and has been used for monitoring levels of contaminating DNA in biopharmaceuticals [65]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA *e.g.* AppTec™ Laboratory Services, BioReliance™, Althea Technologies, *etc.* A comparison of a chemiluminescent hybridisation assay and the total DNA Threshold™ system for measuring host cell DNA contamination of a human viral vaccine can be found in reference 67.

### Pharmaceutical compositions

Vaccines for use with the invention usually include components in addition to the influenza antigens *e.g.* they typically include one or more pharmaceutical carrier(s) and/or excipient(s). A thorough discussion of such components is available in reference 68. In many embodiments adjuvants may also be included.

Compositions will generally be in aqueous form at the point of administration.

A composition may include preservatives such as thiomersal or 2-phenoxyethanol. It is preferred that the vaccine should be substantially free from *(e.g.* <10µg/ml) mercurial material *e.g.* thiomersal-free [14,69]. Vaccines containing no mercury are more preferred, and α-tocopherol succinate can be included as an alternative to mercurial compounds [14]. Preservative-free vaccines are particularly preferred.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate, and/or magnesium chloride, *etc.* Where adjuvant is in a separate container from antigens, sodium chloride may be present in both containers.

Compositions may have an osmolality of between 200 mOsm/kg and 400 mOsm/kg, preferably between 240-360 mOsm/kg, maybe within the range of 290-310 mOsm/kg.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a composition will generally be between 5.0 and 8.1, and more typically between 6.0 and 8.0 *e.g.* 6.5 and 7.5, or between 7.0 and 7.8.

The composition is preferably sterile. The composition is preferably non-pyrogenic e.g. containing <1 EU (endotoxin unit, a standard measure) per dose, and preferably <0.1 EU per dose. The composition is preferably gluten free.

Compositions of the invention may include detergent *e.g.* a polyoxyethylene sorbitan ester surfactant (known as 'Tween'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may include less than 1mg/ml of each of octoxynol-10 and polysorbate 80. Other residual components in trace amounts could be antibiotics *(e.g.* neomycin, kanamycin, polymyxin B). Where adjuvant is in a separate container from antigens, this detergent will usually be present in the antigen-containing container *(e.g.* antigen with polysorbate 80 and Octoxynol 10).

The composition may include material for a single immunisation, or may include material for multiple immunisations (*i.e*. a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume of about 0.5ml, although a half dose *(i.e.* about 0.25ml) may also be administered, particularly to children.

Vaccines are preferably stored at between 2°C and 8°C. They should not be frozen. They should ideally be kept out of direct light.

Vaccines may be supplied in any suitable container, either formulated ready for administration or as a kit of parts for extemporaneous mixing prior to administration e.g. as separate antigen and adjuvant components (as in the PREPANDRIX™ product). Suitable containers include vials, syringes *(e.g.* disposable syringes), nasal sprays, *etc.* These containers should be sterile. Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) *e.g.* 10 doses. Preferred vials are made of colorless glass. A vial can have a cap *(e.g.* a Luer lock) adapted such that a syringe can be inserted into the cap. A vial may have a cap that permits aseptic removal of its contents, particularly for multidose vials. Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber.

Containers may be marked to show a half-dose volume *e.g.* to facilitate delivery to children. For instance, a syringe containing a 0.5ml dose may have a mark showing a 0.25ml volume.

Where a glass container *(e.g.* a syringe or a vial) is used, then it is preferred to use a container made from a borosilicate glass rather than from a soda lime glass.

A container may be packaged *(e.g.* in the same box) with a leaflet including details of the vaccine *e.g.* instructions for administration, details of the antigens within the vaccine, *etc.* The instructions may also contain warnings *e.g.* to keep a solution of adrenaline readily available in case of anaphylactic reaction following vaccination, *etc.*

### Adjuvants

At the point of use, vaccines of the invention may advantageously include an adjuvant, which can function to enhance the immune responses (humoral and/or cellular) elicited in a patient who receives the composition. The presence of an oil-in-water emulsion adjuvant (particularly one comprising squalene) has been shown to enhance the strain cross-reactivity of immune responses for seasonal [70] and pandemic [71,72] influenza vaccines.

Oil-in-water emulsions for use with the invention typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil, *etc.* In the grain group, corn oil is the most readily available, but the oil of other cereal grains such as wheat, oats, rye, rice, teff, triticale, *etc.* may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoid known as squalene, 2,6,10,15,19,23-hexamethyl-2,6,10,14,18,22-tetracosahexaene. Squalane, the saturated analog to squalene, can also be used. Fish oils, including squalene and squalane, are readily available from commercial sources or may be obtained by methods known in the art. Squalene is preferred.

Other useful oils are the tocopherols, which are advantageously included in vaccines for use in elderly patients *(e.g.* aged 60 years or older) because vitamin E has been reported to have a positive effect on the immune response in this patient group [73]. They also have antioxidant properties that may help to stabilize the emulsions [74]. Various tocopherols exist (α, β, γ, δ, ε or ξ) but α is usually used. A preferred α-tocopherol is DL-α-tocopherol. α-tocopherol succinate is known to be compatible with influenza vaccines and to be a useful preservative as an alternative to mercurial compounds [14].

Mixtures of oils can be used *e.g.* squalene and α-tocopherol. An oil content in the range of 2-20% (by volume) is typical.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. The most preferred surfactant for including in the emulsion is polysorbate 80 (polyoxyethylene sorbitan monooleate; Tween 80).

Mixtures of surfactants can be used *e.g.* Tween 80/Span 85 mixtures. A combination of a polyoxyethylene sorbitan ester and an octoxynol is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1%; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Squalene-containing oil-in-water emulsions are preferred, particularly those containing polysorbate 80. Specific oil-in-water emulsion adjuvants useful with the invention include, but are not limited to:
- A submicron emulsion of squalene, polysorbate 80, and sorbitan trioleate. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [75-77], as described in more detail in Chapter 10 of ref. 78 and chapter 12 of ref. 79. The MF59 emulsion advantageously includes citrate ions *e.g.* 10mM sodium citrate buffer.
- A submicron emulsion of squalene, a tocopherol, and polysorbate 80. These emulsions may have from 2 to 10% squalene, from 2 to 10% tocopherol and from 0.3 to 3% polysorbate 80, and the weight ratio of squalene:tocopherol is preferably ≤1 *(e.g.* 0.90) as this can provide a more stable emulsion. Squalene and polysorbate 80 may be present at a volume ratio of about 5:2 or at a weight ratio of about 11:5. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion has submicron oil droplets *e.g.* with an average diameter of between 100 and 250nm, preferably about 180nm. The emulsion may also include a 3-de-O-acylated monophosphoryl lipid A (3d-MPL). Another useful emulsion of this type may comprise, per human dose, 0.5-10 mg squalene, 0.5-11 mg tocopherol, and 0.1-4 mg polysorbate 80 [80].
- An emulsion of squalene, a tocopherol, and a Triton detergent *(e.g.* Triton X-100). The emulsion may also include a 3d-MPL (see below). The emulsion may contain a phosphate buffer.
- An emulsion comprising a polysorbate *(e.g.* polysorbate 80), a Triton detergent *(e.g.* Triton X-100) and a tocopherol *(e.g.* an α-tocopherol succinate). The emulsion may include these three components at a mass ratio of about 75:11:10 *(e.g.* 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL. The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [81] (0.05-1% Thr-MDP, 5% squalane, 2.5% Pluronic L121 and 0.2% polysorbate 80). It can also be used without the Thr-MDP, as in the "AF" adjuvant [82] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant *(e.g.* polyoxyethylene (12) cetostearyl ether) and a hydrophobic nonionic surfactant *(e.g.* a sorbitan ester or mannide ester, such as sorbitan monoleate or 'Span 80'). The emulsion is preferably thermoreversible and/or has at least 90% of the oil droplets (by volume) with a size less than 200 nm [83]. The emulsion may also include one or more of: alditol; a cryoprotective agent *(e.g.* a sugar, such as dodecylmaltoside and/or sucrose); and/or an alkylpolyglycoside. The emulsion may include a TLR4 agonist [84]. Such emulsions may be lyophilized.
- An emulsion of squalene, poloxamer 105 and Abil-Care [85]. The final concentration (weight) of these components in adjuvanted vaccines are 5% squalene, 4% poloxamer 105 (pluronic polyol) and 2% Abil-Care 85 (Bis-PEG/PPG-16/16 PEG/PPG-16/16 dimethicone; caprylic/capric triglyceride).
- An emulsion having from 0.5-50% of an oil, 0.1-10% of a phospholipid, and 0.05-5% of a non-ionic surfactant. As described in reference 86, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 87, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin *(e.g.* QuilA or QS21) and a sterol *(e.g.* a cholesterol) are associated as helical micelles [88].
- An emulsion comprising a mineral oil, a non-ionic lipophilic ethoxylated fatty alcohol, and a non-ionic hydrophilic surfactant *(e.g.* an ethoxylated fatty alcohol and/or polyoxyethylenepolyoxypropylene block copolymer) [89].
- An emulsion comprising a mineral oil, a non-ionic hydrophilic ethoxylated fatty alcohol, and a non-ionic lipophilic surfactant *(e.g.* an ethoxylated fatty alcohol and/or polyoxyethylenepolyoxypropylene block copolymer) [89].

The emulsions may be combined with antigen(s) during vaccine manufacture, or may be supplied as a separate component for mixing with a separate antigen-containing component extemporaneously, at the time of delivery (as in the PREPANDRIX™ product). Where these two components are liquids then the volume ratio of the two liquids for mixing can vary *(e.g.* between 5:1 and 1:5) but is generally about 1:1.

After the antigen and adjuvant have been mixed, haemagglutinin antigen will generally remain in aqueous solution but may distribute itself around the oil/water interface. In general, little if any haemagglutinin will enter the oil phase of the emulsion.

In some embodiments, only one of the first and second vaccines is adjuvanted (preferably the first). Preferably, however, both vaccines are adjuvanted, typically using the same adjuvant.

### Administration of the vaccine,

Vaccines are used in methods of raising an immune response in a patient, comprising the step of administering a composition of the invention to the patient.

Methods, kits and uses of the invention will generally be used to generate an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [90]. Antibody responses are typically measured by hemagglutination inhibition, by microneutralisation, by single radial immunodiffusion (SRID), and/or by single radial hemolysis (SRH). Pseudotype assays can be used. All of these assay techniques are well known in the art.

Compositions of the invention can be administered in various ways. The most preferred immunisation route is by intramuscular injection *(e.g.* into the arm or leg), but other available routes include subcutaneous injection, intranasal [91-93], oral [94], buccal, sublingual, intradermal [95,96], transcutaneous, transdermal [97], *etc.*

Vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly *(e.g.* ≥50 years old, ≥60 years old, and preferably ≥65 years), the young *(e.g.* ≤5 years old), hospitalised patients, healthcare workers, armed service and military personnel, pregnant women, the chronically ill, immunodeficient patients, patients who have taken an antiviral compound *(e.g.* an oseltamivir or zanamivir compound; see below) in the 7 days prior to receiving the vaccine, people with egg allergies and people travelling abroad. The vaccines are not suitable solely for these groups, however, and may be used more generally in a population.

Vaccines may be administered in a pre-pandemic setting (*i.e*. before a pandemic has occurred) or in a pandemic setting (*i.e.* after a pandemic outbreak has started).

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Vaccines produced by the invention may be administered to patients at substantially the same time as *(e.g.* during the same medical consultation or visit to a healthcare professional or vaccination centre) other vaccines *e.g.* at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as *(e.g.* during the same medical consultation or visit to a healthcare professional) an antiviral compound, and in particular an antiviral compound active against influenza virus *(e.g.* oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof *(e.g.* the ethyl esters) and salts thereof *(e.g.* the phosphate salts). A preferred antiviral is (3R,4R,SS)-4-acetylamino-5-amino-3(1-ethylpropoxy)-1-cyclohexene-1-carboxylic acid, ethyl ester, phosphate (1:1), also known as oseltamivir phosphate (TAMIFLU™).

### The regimen

Regimens of the invention involve administration of two doses of pandemic-associated antigen, with the two doses being administered 1 week apart, 2 weeks apart or 6 weeks apart. No pandemic-associated antigen is administered in between these two doses, but further pandemic-associated antigen may be administered after the second dose.

When two doses are given 1 week apart, these may be precisely 7 days apart *(e.g.* on two consecutive Mondays), but according to standard practice there is some leeway. Similarly, doses given 2 or 6 weeks apart are also subject to leeway. Thus two doses may be given between *x* and *y* days apart.

In a first embodiment, the value *of x* is 5 and the value of *y* is 9. In a second embodiment, the value of *x* is 6 and the value of *y* is 8.

In a third embodiment, the value ofx is 12 and the value of *y* is 16. In a fourth embodiment, the value ofx is 13 and the value of *y* is 15.

In a fifth embodiment, the value *of x* is 35 and the value of *y* is 49. In a sixth embodiment, the value *of x* is 38 and the value of *y* is 46. In a seventh embodiment, the value *of x* is 40 and the value of *y* is 44. In an eighth embodiment, the value *of x* is 41 and the value of *y* is 43.

In some embodiments, the values of *x* and *y* are identical, in which case the phrase "between x and *y"* becomes simply *"x",* where *x* is 7, 14 or 42.

### General

The term "comprising" encompasses "including" as well as "consisting" *e.g.* a composition "comprising" X may consist exclusively of X or may include something additional *e.g.* X + Y.

The word "substantially" does not exclude "completely" e.g. a composition which is "substantially free" from Y may be completely free from Y. Where necessary, the word "substantially" may be omitted from the definition of the invention.

The term "about" in relation to a numerical value *x* is optional and means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a cell substrate is used for reassortment or reverse genetics procedures, or for viral growth, it is preferably one that has been approved for use in human vaccine production *e.g.* as in Ph Eur general chapter 5.2.3.

### MODES FOR CARRYING OUT THE INVENTION

A prospective randomized open-label phase III study is performed in approximately 240 subjects aged 18 to 60 years. The subjects are randomized at a 1:1:1:1 ratio and receive one of four different vaccination schedules. Vaccines are administered 1, 2, 3, or 6 weeks apart intramuscularly into the deltoid muscle (preferably of the non-dominant arm). Blood samples are taken from all subjects before each vaccination (visit 1 and 2) and 3 weeks after the second vaccination (visit 3) for the evaluation of immunogenicity by hemagglutination inhibition (HI), microneutralization (MN), and single radial hemolysis (SRH). The blood samples from the 2-week and 3-week groups are further tested for immune responses against a heterologous H5N1 (A/turkey/Turkey/1/05) antigen.

The same vaccine is administered to all patients, namely a 0.5 mL monovalent surface antigen vaccine including 7.5µg hemagglutinin from an A/H5N1 strain. The vaccine is adjuvanted with the MF59™ squalene-in-water emulsion and is made by mixing 0.25 mL of 2x adjuvant with 0.25mL of 2x antigen. The vaccine is supplied in pre-filled syringes with an overfill of up to 0.05 mL.

All the CHMP criteria are met in the groups which received the second vaccine 2, 3 or 6 weeks after the first vaccine, based on MN, HI and SRH assays with a homologous vaccine antigen (NIBRG14; clade 1). The seroconversion rates in the 2-week and 6-week groups are higher (76% and 79%, respectively) following the second dose compared to the 3-week group (72%). Likewise, the percentage of patients which showed a 4-fold increase in the MN assay was higher in the 2-week and 6-week groups (75% and 90% respectively) compared to the 3-week group (73%). The vaccine also induce heterologous immunoprotection against the heterologous A/turkey/Turkey/1/05 as shown by SRH.

The patients in the 1-week, 2-week and 6-week groups also show fewer local adverse events, like pain, redness and swelling in response to the vaccine (see table 1).

**TABLE 1: percentage of patients which show solicited local adverse events**

| | **Post-vaccine 1** | | | | **Post-vaccine 2** | | | |
|---|---|---|---|---|---|---|---|---|
| | **1-Week** | **2-Week** | **3-Week** | **6-Week** | **1-Week** | **2-Week** | **3-Week** | **6-Week** |
| Pain | 73 | 65 | 70 | 63 | 37 | 34 | 48 | 37 |
| Redness | 0- | 0 | 12 | 12 | 0 | 3 | 8 | 5 |
| Swelling | 3 | 2 | 10 | 7 | 0 | 0 | 7 | 5 |

It will be understood that the invention has been described by way of example only and modifications may be made whilst remaining within the scope of the invention.

### REFERENCES

[1] Leroux-Roels et al. (2008) PLoS ONE 3(2): e1665. doi:10.1371/journal.pone.0001665
[2] Bresson et al. (2006) Lancet 367:1657-64.
[3] Levie et al. (2008) J Infect Dis 198:642-9.
[4] Stephenson et al. (2008) NEJM 359:1631-3.
[5] Nolan et al. (2008) Vaccine 26:4160-7.
[6] Ehrlich et al. (2008) NEJM 358:2573-84.
[7] Girard et al. (2008) Vaccine 26:4975-7.
[8] Zangwill et al. (2008) J Infect Dis 197:580-3.
[9] Ruat et al. (2008) J Virol 82:2565-9.
[10] WO02/28422.
[11] WO02/067983.
[12] WO02/074336.
[13] WO01/21151.
[14] WO02/097072.
[15] WO2005/113756.
[16] Huckriede et al. (2003) Methods Enzymol 373:74-91.
[17] de Vries et al. (2009) Vaccine 27:945-55.
[18] Treanor et al. (1996) J Infect Dis 173:1467-70.
[19] Keitel et al. (1996) Clin Diagn Lab Immunol 3:507-10.
[20] WO 2008/068631.
[21] Sandbulte et al. (2007) PLoS Med. 4(2):e59.
[22] Hoffmann et al. (2002) Vaccine 20:3165-3170.
[23] Subbarao et al. (2003) Virology 305:192-200.
[24] Liu et al. (2003) Virology 314:580-590.
[25] Ozaki et al. (2004) J. Virol. 78:1851-1857.
[26] Webby et al. (2004) Lancet 363:1099-1103.
[27] WO00/60050.
[28] WO01/04333.
[29] US patent 6649372.
[30] Neumann et al. (2005) Proc Nntl Acad Sci USA 102:16825-9.
[31] WO2006/067211.
[32] WO01/83794.
[33] Hoffmann et al. (2000) Virology 267(2):310-7.
[34] Herlocher et al. (2004) J Infect Dis 190(9):1627-30.
[35] Le et al. (2005) Nature 437(7062):1108.
[36] Gambaryan & Matrosovich (1992) J Virol Methods 39(1-2):111-23.
[37] Mastrosovich et al. (1999) J Virol 73: 1146-55.
[38] Stevens et al. (2006) J Mol Biol 355:1143-55.
[39] Couceiro & Baum (1994) Mem Inst Oswaldo Cruz 89(4):587-91.
[40] Kistner et al. (1998) Vaccine 16:960-8.
[41] Kistner et al. (1999) Dev Biol Stand 98:101-110.
[42] Bruhl et al. (2000) Vaccine 19:1149-58.
[43] Pau et al. (2001) Vaccine 19:2716-21.
[44] *http:*//*www.atcc.org*/
[45] *http:*//*locus.umdnj.edu*/
[46] WO03/076601.
[47] WO2005/042728.
[48] WO03/043415.
[49] WO01/85938.
[50] WO2006/108846.
[51] WO97/37000.
[52] Brands et al. (1999) Dev Biol Stand 98:93-100.
[53] Halperin et al. (2002) Vaccine 20:1240-7.
[54] Tree et al. (2001) Vaccine 19:3444-50.
[55] EP-A-1260581 (WO01/64846).
[56] WO2006/071563.
[57] WO2005/113758.
[58] WO97/37001.
[59] WO2006/027698.
[60] EP-B-0870508.
[61] US 5948410.
[62] Lundblad (2001) Biotechnology and Applied Biochemistry 34:195-197.
[63] Guidance for Industry: Bioanalytical Method Validation. U.S. Department of Health and Human Services Food and Drug Administration Center for Drug Evaluation and Research (CDER) Center for Veterinary Medicine (CVM). May 2001.
[64] Ji et al. (2002) Biotechniques. 32:1162-7.
[65] Briggs (1991) J Parenter Sci Technol. 45:7-12.
[66] Lahijani et al. (1998) Hum Gene Ther. 9:1173-80.
[67] Lokteff et al. (2001) Biologicals. 29:123-32.
[68] Gennaro (2000) Remington: The Science and Practice of Pharmacy. 20th edition, ISBN: 0683306472.
[69] Banzhoff (2000) Immunology Letters 71:91-96.
[70] O'Hagan (2007) Expert Rev Vaccines. 6(5):699-710.
[71] Bernstein et al. (2008) J Infect Dis. 197(5):667-75.
[72] Stephenson et al. (2005) J Infect Dis. 191(8):1210-5.
[73] Han et al. (2005) Impact of Vitamin E on Immune Function and Infections Diseases in the Aged at Nutrition, Immune functions and Health EuroConference, Paris, 9-10 June 2005.
[74] US-6630161.
[75] WO90/14837.
[76] Podda & Del Giudice (2003) Expert Rev Vaccines 2:197-203.
[77] Podda (2001) Vaccine 19: 2673-2680.
*[78]* Vaccine Design: The Subunit and Adjuvant Approach (eds. Powell & Newman) Plenum Press 1995 (ISBN 0-306-44867-X).
*[79]* Vaccine Adjuvants: Preparation Methods and Research Protocols (Volume 42 of Methods in Molecular Medicine series). ISBN: 1-59259-083-7. Ed. O'Hagan.
[80] WO2008/043774.
[81] Allison & Byars (1992) Res Immunol 143:519-25.
[82] Hariharan et al. (1995) Cancer Res 55:3486-9.
[83] US-2007/0014805.
[84] US-2007/0191314.
[85] Suli et al. (2004) Vaccine 22(25-26):3464-9.
[86] WO95/11700.
[87] US patent 6,080,725.
[88] WO2005/097181.
[89] WO2006/113373.
[90] Potter & Oxford (1979) Br Med Bull 35: 69-75.
[91] Greenbaum et al. (2004) Vaccine 22:2566-77.
[92] Zurbriggen et al. (2003) Expert Rev Vaccines 2:295-304.
[93] Piascik (2003) J Am Pharm Assoc (Wash DC). 43:728-30.
[94] Mann et al. (2004) Vaccine 22:2425-9.
[95] Halperin et al. (1979) Am JPublic Health 69:1247-50.
[96] Herbert et al. (1979) J Infect Dis 140:234-8.
[97] Chen et al. (2003) Vaccine 21:2830-6.

## Claims

1. First and second influenza vaccines comprising antigen from a pandemic-associated influenza virus strain, for use in immunizing a human by a method comprising steps of: (a) administering to the human a first vaccine comprising antigen from a pandemic-associated influenza virus strain; and then w week(s) later, (b) administering to the same human a second influenza vaccine comprising antigen from the pandemic-associated influenza virus strain ... wherein w is 1, 2 or 6.

2. The use of first and second vaccines, each comprising antigen from the same pandemic-associated influenza virus strain, in the manufacture of a medicament for use in immunizing a human, wherein the first and second vaccines are administered to the same human w week(s) apart ... wherein w is 1, 2 or 6.

3. The use of:
(i) a first vaccine comprising antigen from a pandemic-associated influenza virus strain, in the manufacture of a medicament for pre-immunizing a human who will receive a second vaccine comprising antigen from the pandemic-associated influenza virus strain w week(s) later; or
(ii) a second vaccine comprising antigen from a pandemic-associated influenza virus strain, in the manufacture of a medicament for immunizing a human who had received a first vaccine comprising antigen from the pandemic-associated influenza virus strain w week(s) before receiving the second vaccine;
wherein w is 1, 2 or 6.

4. A kit comprising first and second vaccines, each comprising antigen from the same pandemic-associated influenza virus strain, wherein the first and second vaccines are for administration to a human w week(s) apart ... wherein w is 1, 2 or 6.

5. The vaccines, use or kit of any preceding claim, wherein the first and second vaccines are both inactivated virus vaccines.

6. The vaccines, use or kit of claim 5, wherein the first and second vaccines are both split virion vaccines or are both purified surface antigen vaccines.

7. The vaccines, use or kit of any preceding claim, wherein the pandemic-associated influenza virus strain has a H5 hemagglutinin subtype *(e.g.* H5N1) or a H1 hemagglutinin subtype *(e.g.* H1N1).

8. The vaccines, use or kit of any preceding claim, wherein the first and second vaccines include between 0.1 and 50 µg of hemagglutinin e.g. about 15 µg, about 10 µg, about 7.5 µg, about 5 µg, about 3.75 µg, about 1.9 µg, or about 1.5 µg.

9. The vaccines, use or kit of any preceding claim, wherein the first and second vaccines are adjuvanted with an oil-in-water emulsion adjuvant comprising squalene.

10. The vaccines, use or kit of claim 9, wherein the emulsion has submicron oil droplets and comprises (i) squalene, polysorbate 80, and sorbitan trioleate or (ii) squalene, DL-α-tocopherol, and polysorbate 80 or (iii) comprises squalene, an aqueous solvent, a polyoxyethylene alkyl ether hydrophilic nonionic surfactant and a hydrophobic nonionic surfactant.

11. The vaccines, use or kit of any preceding claim, wherein antigen from the pandemic-associated influenza virus strain is the only antigen in a vaccine.

12. The vaccines, use or kit of any preceding claim, wherein the first and second vaccines are both thiomersal-free.

13. The vaccines, use or kit of any preceding claim, wherein the first and second vaccines are administered in a dosage volume of about 0.5ml.

14. The vaccines, use or kit of any preceding claim, wherein the first and second vaccines are administered by intramuscular injection.
